Europäisches Patentamt

European Patent Office

Office européen des brevets

(11) Publication number: **0 445 660 A2**

# EUROPEAN PATENT APPLICATION

(21) Application number: **91103053.4**

(22) Date of filing: **28.02.91**

(51) Int. Cl.5: **C07C 29/152, F01K 23/00**

(30) Priority: **07.03.90 US 489985**

(43) Date of publication of application:
**11.09.91 Bulletin 91/37**

(84) Designated Contracting States:
**BE DE DK ES FR GB IT NL SE**

(71) Applicant: **AIR PRODUCTS AND CHEMICALS, INC.**
**7201 Hamilton Boulevard**
**Allentown, PA 18195-1501(US)**

(72) Inventor: **Hsiung, Thomas Hsiao-Long**
**4727 Glenwood Circle**
**Emmaus, PA 18049(US)**
Inventor: **Perka, Alan Thomas**
**1701 John B. Dennis Highway No.40**
**Kingsport, TN 37664(US)**
Inventor: **Klosek, Joseph**
**1416 Mashie Drive**
**Wescosville, PA 18106(US)**
Inventor: **Moore, Robert Byron**
**2951 Edgemont Court**
**Allentown, PA 18103(US)**

(74) Representative: **Kador & Partner**
**Corneliusstrasse 15**
**W-8000 München 5(DE)**

(54) Integrated production of methanol and electric power.

(57) A method is disclosed for the production of methanol and fuel gas from CO-rich synthesis gas and water in a liquid phase reactor containing a solid catalyst suspended in a paraffinic liquid. The addition of water in appropriate amounts increases the reactor methanol productivity compared with that obtained without the addition of water. The method can be integrated with an Integrated Gasification Combined Cycle (IGCC) power generation process to coproduce methanol and electric power.

FIG.1

EP 0 445 660 A2

The present application is a Continuation-in-Part of U. S. Serial No. 07/178,955 filed April 7, 1988.

TECHNICAL FIELD

The present invention relates to a method for the coproduction of methanol and electric power by the integration of a liquid phase methanol reactor with an Integrated Gasification Combined Cycle (IGCC) power generation process.

BACKGROUND OF THE INVENTION

Methanol is produced from synthesis gas (syngas), a mixture of hydrogen ($H_2$), carbon monoxide (CO), and carbon dioxide ($CO_2$). The stoichiometry of the methanol synthesis reactions indicates that the desired molar reactor feed composition is given by the equation:

$$R = (H_2 - CO_2)/(CO + CO_2) = 2.0$$

However, reaction kinetics and system control dictate that the optimum ratio is actually $R = 2.1$ or higher. Gas with $R = 2.0$ to 2.1 is called "balanced" gas, i.e. balanced stoichiometrically, and has a typical composition of 19% CO, 5% $CO_2$, 55% $H_2$, and 21% $CH_4$-$N_2$.

Syngas is commonly made by the reforming of methane or other hydrocarbons, which gives a hydrogen-rich gas well-suited for methanol synthesis (e.g., a typical methanol syngas produced by steam reforming of methane has a composition of 15% CO, 8% $CO_2$, 73% $H_2$, 4% $CH_4$-$N_2$, $R = 2.8$). Currently 70 to 75% of the world's methanol comes from reformed natural gas, however, because of the instability of the oil market, liquid hydrocarbons and natural gas are not always readily available or available at an inexpensive cost. An alternative and abundant resource is coal, which can be converted to syngas in a coal gasifier such as the advanced, high-temperature coal gasifiers developed by Texaco, Dow, Shell, and British Gas/Lurgi, or the well-established, older coal gasifiers developed by Lurgi and Krupp-Koppers.

Coal-derived syngas can be used as gas turbine fuel in an integrated gasification combined cycle (IGCC) electric power plant. Because of the daily cyclical demand for power, a primary concern in such a facility is load-following flexibility. To accomplish this flexibility, either the front end of the IGCC plant must be built for peak capacity, or extra fuel must be imported during peak periods (called peak shaving). The former is an expensive and inefficient option. The latter, although somewhat less expensive, can be improved by producing and storing the fuel on-site. One solution to this problem is the on-site production of methanol as the peak-shaving fuel.

In an IGCC facility without methanol coproduction, the syngas is combusted in a gas turbine to produce electricity. The turbine exhaust/stack gas is used to generate and superheat steam in an integrated heat recovery system, and this steam is also used to generate electricity. In a coproduction facility, the syngas is first passed through a methanol synthesis reactor to convert a portion to methanol; the remaining syngas is fed to the gas turbine for power production. The methanol is stored as peak-shaving fuel, which is used to augment the feed to the gas turbine during periods of high power demand. This scheme is attractive because the load on a power plant varies over a wide range, and it is more economical to feed the stored methanol than to build peak-shaving capacity into the front end of the facility.

Unfortunately, coal-derived syngas from high-temperature gasifiers used in IGCC plants is CO-rich (e.g., a Texaco gasifier syngas has a typical composition of 35% $H_2$, 51% CO, 13% $CO_2$, 1% $CH_4$-$N_2$; $R = 0.34$), unlike the hydrogen-rich syngas from reformed hydrocarbons. The problem is that converting this gas to methanol by conventional methods is expensive and complicated because several pretreatment steps are required to balance the gas prior to methanol synthesis.

Conceptual IGCC coproduct plants have been designed with gas-phase and with liquid-phase methanol synthesis reactors. With a gas-phase reactor, the main syngas stream from the gasifier is divided into two parts: approximately 75% goes directly to the gas turbine, and the remaining 25% goes to the methanol synthesis section. This latter stream is further divided, approximately 67% being mixed with steam and sent to a high temperature shift reactor (HTS). After shift, the $CO_2$ is removed and this stream is remixed with the unshifted stream and recycle gas in the methanol loop to give a balanced gas for methanol synthesis. Recycle of syngas is required within the methanol synthesis loop to achieve 95-98% conversion of the remaining 25% of the main syngas stream. Purge gas from the recycle loop and the rejected $CO_2$ from the $CO_2$ removal section are sent to the gas turbine. The use of a conventional, gas-phase methanol synthesis reactor in an IGCC coproduct scheme is subject to the same shortcomings as in a gas-phase all-methanol product plant: a shift section and $CO_2$ removal section are required in order to achieve a feed gas

2

composition with an "R" value greater than 2.0, shift and methanol synthesis are performed in separate vessels, and the conversion per pass is limited by temperature constraints.

The liquid-phase methanol process has an advantage over gas-phase methanol synthesis in a coproduct configuration because of its ability to directly process CO-rich gas (e.g., "R" values between about 0.30 and 0.40). The entire CO-rich gas stream from the gasifier is sent through the liquid-phase reactor in a single pass, achieving 10-20% conversion of CO to methanol, which is equivalent to about 18-36% of the BTU content of the gas. While additional methanol can be produced by balancing the gas prior to feeding it to the liquid-phase methanol reactor, the value of this incremental methanol is outweighed by the cost of separate shift and $CO_2$ removal units. Because a liquid-phase methanol reactor operates isothermally, there is no increasing catalyst temperature and the accompanying constraint on methanol conversion which is characteristic of gas-phase methanol synthesis processes. In a typical liquid-phase design, approximately 14% of the CO (feedgas "R" = 0.34) is converted to methanol (equivalent to 25% of the BTU content of the gas), giving a reactor effluent containing approximately 9% methanol; the per pass conversion in a gas-phase reactor generally results in a reactor effluent containing only 5% methanol even though the feedgas has an "R" greater than 2.0. Therefore, recycle is practiced to increase the absolute conversion to methanol. It should be noted, however, that even with the superior performance of the liquid-phase reactor, the coproduction scheme can still be expensive, and there is incentive to improve this processing route.

A somewhat similar coproduction scheme is also worthy of mention (U.S. Pat. 3,986,349 and 4,092,825). This scheme involves converting coal-derived syngas into liquid hydrocarbons via Fischer-Tropsch synthesis, separating the liquid hydrocarbons from the unreacted gas, feeding the gas to a gas turbine to generate electric power, and using at least part of the hydrocarbons as peak-shaving fuel. Although methanol is mentioned as a possible by-product of the hydrocarbon synthesis, it is not one of the desired products.

U. S. Patent 2,665,289 discloses a process in which CO-rich synthesis gas and water vapor are contacted with an iron-containing hydrocarbon synthesis catalyst in a gas-phase fluidized bed reactor to yield hydrocarbons, oxygenated hydrocarbons, and mixtures thereof. A portion of the unreacted synthesis gas is treated to remove $CO_2$ and the resulting gas is recycled back to the reactor.

German Patent Application No. 1,300,538 and U.S.S.R. Patent Application No.1154413/23-4 disclose the synthesis of methanol from hydrogen and CO in a gas-phase reactor containing a copper/zinc/chromium oxide catalyst. Water vapor is added to the reactor feed to improve the activity of the catalyst and the yield of methanol.

The preparation of methanol from hydrogen and carbon oxides is described in U. S. Patent 4,031,123 in which a feed gas is passed through a catalyst bed in contact with an inert paraffinic or cycloparaffinic hydrocarbon liquid. The equivalent methanol concentration in the liquid is less than 1.0 wt%.

A report by M. Sherwin and D. Blum entitled "Liquid Phase Methanol", Report No. AF-1291 prepared for Research Project 317-2 for the Electric Power Research Institute, December 1979, at p. 5-3 describes the addition of steam to CO-rich synthesis gas feed to a liquid phase reactor containing an unnamed commercial methanol catalyst and Exxon Aromatic 150, an aromatic hydrocarbon liquid. The addition of steam was found to decrease the reactor methanol productivity relative to the productivity without steam.

European Patent Publication No. 0 035 328 discloses a method of making hydrocarbons and alcohols by contacting a gaseous mixture of steam, hydrogen, and CO with a hydrided titanium catalyst in a gas-phase packed bed reactor.

The synthesis of hydrocarbons and oxygenated hydrocarbons including methanol from CO-rich synthesis gas in a conventional gas-phase packed-bed reactor is disclosed in European Patent Publication No. 0 047 596. In one embodiment, the shift reactor is reduced or eliminated by adding steam to the reactor feed whereby shift and methanol synthesis take place in the synthesis reactor.

SUMMARY OF THE INVENTION

The present invention is a method for producing methanol and fuel gas from carbon monoxide-rich synthesis gas. The synthesis gas and water are introduced into a liquid phase reactor containing a solid catalyst and an inert paraffinic hydrocarbon liquid, the synthesis gas and water react in the presence of the catalyst, and a reactor effluent stream is withdrawn which comprises methanol, hydrogen, carbon monoxide, and carbon dioxide. The reactor effluent stream is cooled and separated into a methanol liquid product and a fuel gas comprising hydrogen, carbon monoxide, and carbon dioxide. The addition of water to the reactor allows both the methanol synthesis and water gas shift reactions to take place simultaneously therein. Water is added to the reactor such that the molar ratio of water to carbon monoxide introduced into the reactor is

3

greater than zero and less than about 0.3, which increases methanol productivity over that obtained when no water is added.

The methanol product and electric power can be coproduced in an integrated process by the additional steps of (a) generating steam by cooling the reactor effluent stream with water by indirect heat exchange; (b) combusting the fuel gas with compressed air in a combustor of a gas turbine; (c) injecting at least a portion of the steam into the combustor; (d) withdrawing hot gas from the combustor and expanding it through the gas turbine; and (e) utilizing the gas turbine to drive an electric generator to generate electric power. At least a portion of the methanol product optionally can be introduced into the combustor with additional compressed air to generate additional electric power during periods of peak power demand.

## BRIEF DESCRIPTION OF THE DRAWINGS

Figure 1 is a plot showing the effect of water, expressed as molar $H_2O/CO$ ratio of the gas entering the liquid-phase methanol reactor, on methanol productivity and on the hydrogen content of the gas leaving the liquid-phase methanol reactor.

Figure 2 is a schematic diagram of an embodiment of the methanol synthesis and combustion turbine sections of an IGCC power plant according to the present invention.

Figure 3 is a plot of methanol productivity for a typical liquid-phase run without water addition.

Figure 4 is a plot of methanol productivity for a run with intermittent water addition.

Figures 5 and 6 are block flow diagrams for a simple once-through liquid-phase methanol IGCC process. Figure 5 shows the process without water addition and Figure 6 with water addition.

Figures 7 and 8 are block flow diagrams for a liquid-phase methanol IGCC process with a membrane recycle. Figure 7 shows the process without water addition and Figure 8 with water addition.

Figure 9 is a comparison of methanol productivity vs molar $H_2/CO$ ratio for a paraffinic liquid and an aromatic liquid used in a liquid phase reactor.

## DETAILED DESCRIPTION OF THE INVENTION

The present invention is an improved methanol production step within an integrated gasification combined cycle process wherein methanol is produced for peak-shaving from CO-rich synthesis gas. CO-rich synthesis gas is defined herein as having the molar ratio "R", defined as $(H_2-CO_2)/(CO+CO_2)$, less than about 2.0. The improvement to the process is the combination of the methanol synthesis and water-gas shift reactions in a single step in order to increase methanol productivity. The improvement of the present invention replaces the need to balance the synthesis gas in shift and $CO_2$ removal steps prior to its conversion to methanol as would be required if a gas-phase methanol synthesis process were used. The present invention is based on the fact that if water is added to the CO-rich syngas feed to a liquid-phase methanol reactor, the water-gas shift and methanol synthesis reactions will take place simultaneously. In fact, if no water is added the reverse water-gas shift reaction is known to take place in either liquid or gas-phase reactors. The addition of water forces the equilibrium in the forward direction (i.e., $CO + H_2O \rightarrow H_2 + CO_2$), and simultaneously improves methanol productivity over that when no water is added.

Several advantages of the liquid-phase methanol reactor have already been mentioned. An additional advantage is seen when considering water addition. In contrast to conventional technologies, liquid water can be added directly to the liquid-phase reactor. This saves the cost of generating high-pressure process steam, and also reduces the net heat which must be removed from the reactor. A conventional gas-phase reactor cannot accept a liquid water feed because thermal shock and rapid vaporization can break up and destroy the catalyst tablets. In addition, water vapor which is added must be kept well above its dew point to prevent condensation and subsequent quenching of the bed due to its plug flow operation.

The addition of steam to a liquid-phase methanol reactor containing an aromatic liquid medium was considered in EPRI Report AF-1291 (December 1979, p. 5-3), wherein the concept is discussed, and laboratory data is presented for two syngas compositions; the data indicated that methanol productivity decreases as water is added. It was reported that water addition always reduces methanol productivity, especially for gases that already have the required $H_2/CO$ stoichiometry, and that for non-stoichiometric synthesis gases, the fall off in productivity with increasing steam/CO ratio is slower.

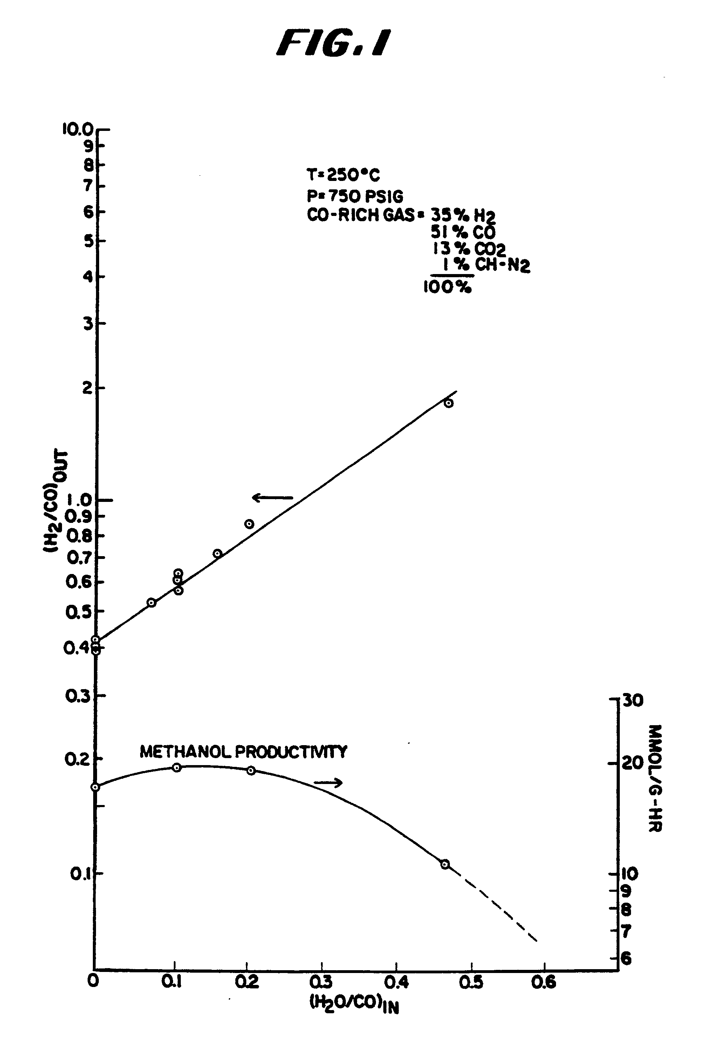

The experimentation behind the present invention, on the other hand, shows results which are surprising relative to those in the EPRI report. Figure 1 shows the effect of water, expressed as the molar $H_2O/CO$ ratio of the gas entering the liquid-phase methanol reactor, on methanol productivity (mmol MeOH/hr-gm catalyst) and on the molar $H_2/CO$ ratio (a measurement of the extent of the water-gas shift reaction) of the gas leaving the liquid-phase methanol reactor. This graph illustrates two important points. First, the methanol productivity curve goes through a maximum, showing that water indeed can be used to boost methanol productivity in addition to eliminating the need for a separate shift reactor. This maximum was not seen or even suspected in the data reported in EPRI Report AF-1291. Second, adding water increases the hydrogen content in the effluent. Although the $CO_2$ produced from the shift reaction prevents a stoichiometrically balanced effluent, the proper amount of $CO_2$ can be removed later to give a balanced gas, if desired. Thus, adding a precise amount of water results in increased methanol production relative to dry CO-rich gas feed as well as a notable production of $H_2$ via the shift reaction. Adding more water results in increased $H_2$ production at some sacrifice to methanol productivity. The increased hydrogen content in the reactor effluent can result in additional methanol production through a second stage of methanol synthesis, or through the recovery and recycle of hydrogen to the single stage methanol synthesis reactor. Another option would be to separate the hydrogen as a byproduct, and also produce methanol and electric power.

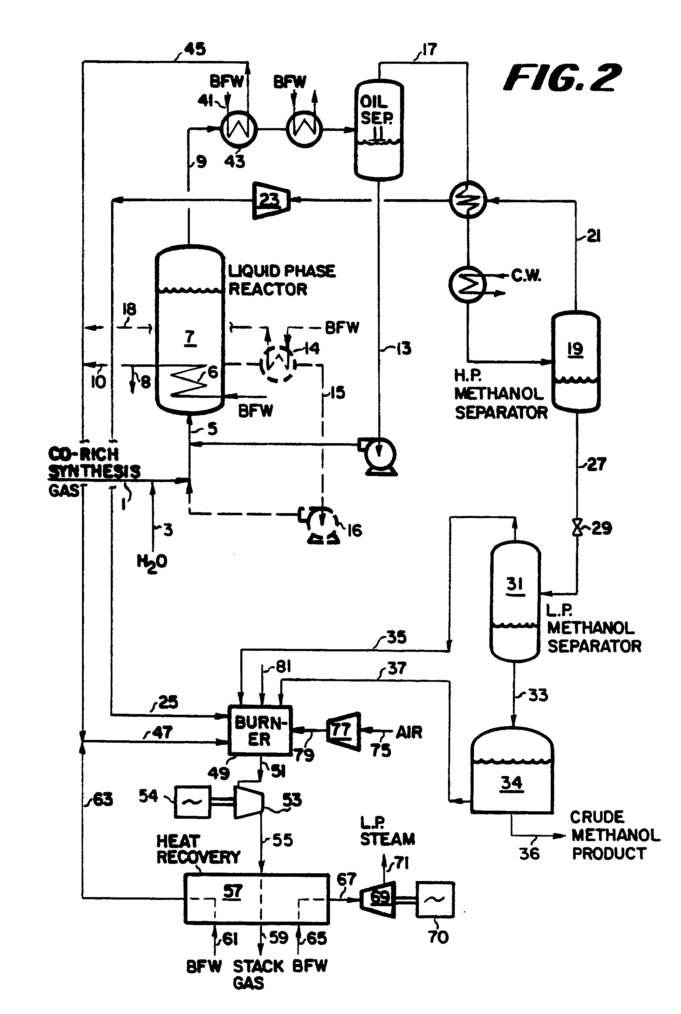

The proposed IGCC plant flowsheet for the coproduction of methanol and electric power according to the present invention is shown in Figure 2. With reference to Figure 2, desulfurized CO-rich synthesis gas and water (liquid or vapor) are fed to the process via lines 1 and 3, respectively, combined, and fed to liquid-phase reactor 7 via line 5, wherein the synthesis gas and water react in the presence of a catalyst. Alternatively, the liquid water or steam, in line 3, can be added directly to reactor 7 without first being combined with the synthesis gas. Liquid-phase methanol reactor 7 can be operated in either a slurry or ebullated mode. In the case of the slurry mode, a powdered methanol synthesis catalyst (e.g., $CuO/ZnO/Al_2O_3$) is slurried in a liquid medium (e.g., light paraffinic or cycloparaffinic oils). Alternatively, a mixture of powdered methanol synthesis catalyst and low temperature shift catalyst can be used in reactor 7. The concentration of catalyst can range from about 5 to 50 wt%. In the case of an ebullated mode, a granulated catalyst is fluidized in a liquid medium. Liquid-phase reactor 7 operates within the conventional understanding of a liquid-phase reactor.

Preferred paraffinic oils can be selected from a number of commercially available oils, several of which are listed along with their properties in Table I.

## TABLE I

### PROPERTIES OF PARAFFINIC PROCESS LIQUIDS

|  | | Witco | | | Penreco | | |
|---|---|---|---|---|---|---|---|
|  | Freezene-100 | Witco-40 | Witco-70 | LP-150 | Drakeol 7 | Drakeol 10 | Multitherm PG1 |
| Average Molecular Weight | 340 | 268 |  |  | 323 | 366 | 350 |
| Distillation (ASTM D1160), °C | | | | | | | |
| IBP | 263 | 268 | 310 | 348 | 316 | 283 | 316 |
| 10% | 317 | 294 | 338 | 379 | 328 | 353 | |
| 50% | 369 | 318 | 363 | 390 | 374 | 407 | |
| 90% | 428 | 341 | 396 | 430 | 449 | 481 | |
| EP | 473 | 380 | 419 | 447 | 512 | | |
| Specific Gravity, g/ml | | | | | | | |
| 25°C | 0.868 | 0.815 | 0.845 | 0.866 | 0.838 | 0.849 | 0.868 |
| 70°C | 0.854 | 0.798 | 0.830 | 0.852 | | | |
| Hydrocarbon Type (PONA) % Paraffinic/Naphthenic | 100 | 100 | 100 | 100 | 66/34 | 65/35 | 86.1/13.9 |
| Surface Tension, DYNE/CM | | | | | | | |
| 25°C | 30.2 | 28.2 | 29.6 | 30.4 | 29.5 | ~30 | 28.0 |
| 250°C | 16.5 | 13 | | | | | |
| Viscosity, cP | | | | | | | |
| 25°C | 34.0 | 6.0 | 19.5 | 57.0 | 17.9 | 31.2 | 30 |
| 75°C | 5.7 | <1 | 4.7 | 8.1 | | | |
| Pour Point, °C | -38 | +2 | -14 | -32 | -7 | -7 | -40 |
| Flash Point, °C | >135 | >174 | >110 | >110 | 179 | 185 | 171 |
| Vapor Pressure, psia 250°C | 1.9 | | | | | | |

The effluent removed via line 9 from liquid-phase reactor 7 is cooled in a series of heat exchangers, including heat exchanger 43, and subsequently separated in separator 11 into a liquid and vapor stream. The primary purpose of separator 11 is to recover and recycle the liquid medium which was vaporized and entrained in the reactor effluent. The liquid stream is recycled via line 13 to liquid-phase reactor 7. Heat is removed from reactor 7 by passing a stream of boiler feedwater through cooling coil 6 to generate steam, which can be taken elsewhere in the process through line 8 and/or sent to burner 49 via lines 10 and 47. Alternately, to provide heat removal from reactor 7, a slurry stream can be removed from the reactor, cooled by heat exchange with boiler feedwater in exchanger 14, and returned to reactor 7 via line 15 and pump 16. The steam generated in this cooling step can be sent to burner 49 via lines 18 and 47.

The vapor stream from oil separator 11 is removed via line 17, cooled in a series of heat exchangers so as to condense methanol and water in the stream and then fed to high pressure methanol separator 19. The overhead from separator 19 is removed via line 21; this overhead is mainly unreacted synthesis gas, which is then reduced in pressure in expander 23 to recover power and subsequently fed as fuel gas to burner 49 via line 25.

The liquid phase from separator 19 is removed via line 27, reduced in pressure in J-T valve 29 and fed to low pressure methanol separator 31. In separator 31, dissolved synthesis gas in the methanol and water solution is removed as overhead via line 35 and fed as fuel gas to burner 49 or used as fuel elsewhere. The bottoms of separator 31 is removed via line 33 to storage tank 34, and crude methanol product is withdrawn therefrom in line 36. This methanol product can be used for transportation fuel, peaking turbine fuel, or after further purification for chemical grade methanol.

The above is a description of a once through methanol synthesis portion of an IGCC process. The combustion portion of the IGCC cycle is as follows: As mentioned earlier, the unreacted synthesis gas from the methanol synthesis portion is fed as fuel gas to burner 49 via lines 25 and 35. These streams are combusted in burner 49 optionally along with fuel gas produced from the sulfur removal step of the gasifier portion of an IGCC facility (fed via line 81), compressed air, and steam. The air is introduced to the process via line 75, compressed in compressor 77 and introduced into the burner via line 79. Steam is produced and introduced into the burner through two heat sources. First, boiler feed water, in line 41, is heated in heat exchanger 43 against the effluent, line 9, from liquid-phase reactor 7 producing steam in line 45. Second, boiler feed water, in line 61, is heated in heat recovery unit 57 producing steam in line 63. These two steam streams, lines 45 and 63, are combined into stream 47 which is then fed to burner 49.

The combustion gas from burner 49 is fed to gas turbine expander 53 via line 51 for recovery of power which is used to drive electric generator 54. Exhaust gas from turbine 53 is fed to heat recovery unit 57 via line 55. In heat recovery unit 57, energy is recovered from the expanded combustion gas by producing steam and superheating steam by heat exchange of the combustion gas with boiler feed water and saturated steam. A portion of the steam produced in heat recovery unit 57 is introduced as feed to burner 49. The remaining portion of steam, in line 67, which is produced from boiler feed water introduced via line 65, is expanded in turbine 69 producing both power and low pressure steam as stream 71; the power is used to drive electric generator 70.

In the above description, stream 1 represents desulfurized CO-rich gas from a Texaco coal gasifier; stream 3 can be used to supply water such that the combined streams (line 5) have a molar ratio of $H_2O/CO$ greater than zero and less than about 0.3, preferably greater than zero and less than about 0.2. As shown in Figure 1, this ratio yields improved methanol productivity over that achieved when no water is added. Stream 5 is fed to liquid-phase reactor 7, which typically operates at about 482° F and 910 psia. The reactor is cooled by passing boiler fed water through cooling coil 6 as earlier described. Optionally, reaction heat is removed in an external heat exchange loop which produces saturated steam as earlier described. The reactor effluent is cooled by first producing steam, then by heat exchange with unreacted fuel gas, and finally with cooling water. The two-phase mixture is separated and the vapor is heated and expanded, producing electric power. This expanded fuel gas is then sent to the gas turbine burner. The condensed methanol is flashed to yield the crude methanol product and a residual gas stream which is also fed to the gas turbine burner or is burned elsewhere as fuel. In addition to the main fuel gas and flash gas streams, the gas turbine burner optionally receives a fuel gas stream from the upstream sulfur removal plant (e.g., Selexol, Rectisol, Rectisol II), sufficient steam from the process to control $NO_x$ production, and compressed air. These streams are fed to the combustion zone, which typically operates at 2300° F. The burner effluent expands across the gas turbine expander, which produces electric power for export and for running the air compressor. The gas turbine exhaust is used to produce and superheat steam in an integrated heat recovery system. The steam subsequently powers steam turbines which produce additional electric power.

An IGCC coproduct plant has two principal modes of operation. During peak power demand times, all of the fuel gas and some stored methanol go to the gas turbine. During off-peak hours, gas flows through the liquid-phase reactor to convert a portion of the gas to methanol for storage. With water addition, the methanol productivity per mass of catalyst is increased, which means that either the reactor can be downsized or additional methanol can be produced from a base-size unit. The plant has greater flexibility because it can operate in three modes: all fuel gas to the gas turbine, gas through the liquid-phase reactor without water addition, and gas through liquid-phase reactor with water added.

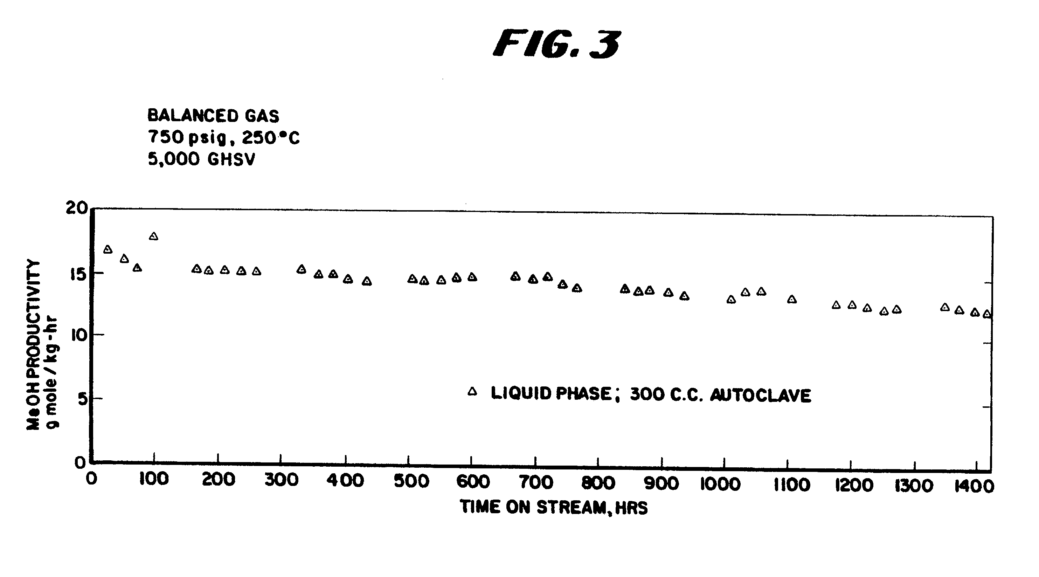

An additional, surprising benefit of water addition has been demonstrated in the laboratory. Figure 3 shows methanol productivity for a typical liquid-phase run with a balanced syngas without water addition. Productivity falls off with time onstream from around 17 to 12.5 gmole/hr-kg. Figure 3 illustrates the expected and well-known fact that methanol synthesis catalyst deactivates with time. Figure 3 also illustrates a characteristic of methanol synthesis catalyst life curves, in that there is an early period of hyperactivity during which the catalyst deactivates sharply: after this hyperactivity period the catalyst deactivates slowly.

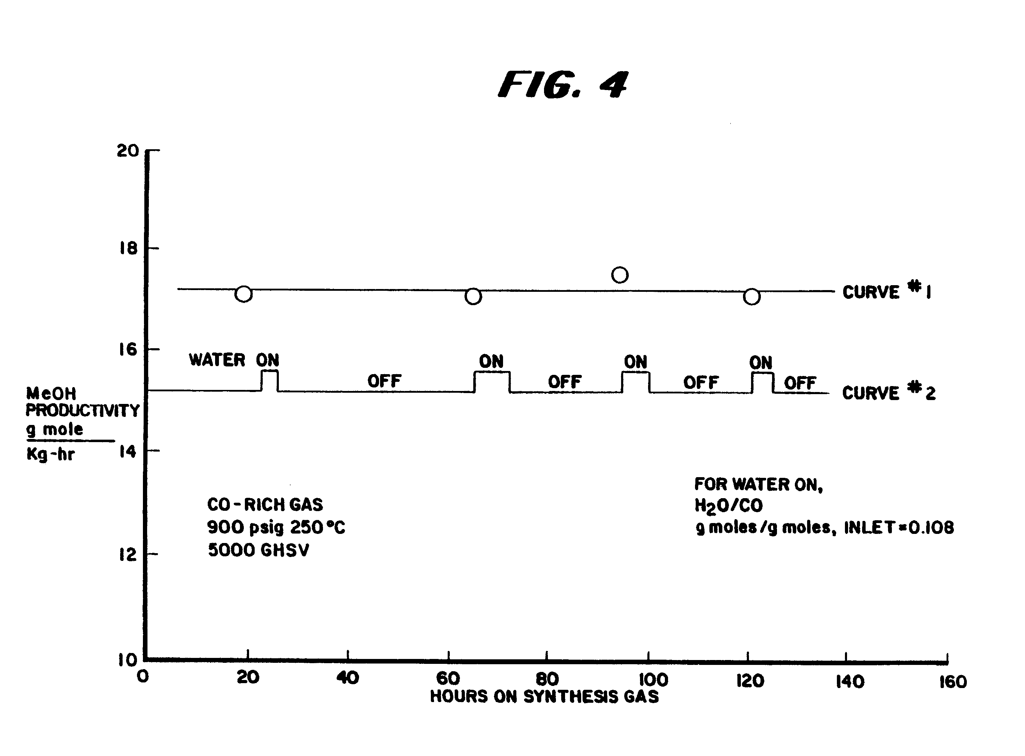

Figure 4 shows methanol productivity for a run with CO-rich syngas and intermittent water addition. Curve #1 shows the baseline methanol productivity trend when water is added as indicated by curve #2. The data points represent the methanol productivity during the periods without water addition; the productivity during periods with water addition always exceed the baseline curve #1. The important point here is that curve #1 is flat, rather than downward sloping, indicating that methanol productivity is not decreasing as was seen in Figure 3. This is especially notable because the comparison is made during the hyperactivity period, when the rate of deactivation is most pronounced. Therefore, Figure 4 indicates that the methanol productivity of the catalyst is preserved by the intermittent addition of water. Thus, the IGCC coproduction plant with water addition not only gets an additional degree of flexibility and a smaller reactor or incremental methanol production, but also a longer-lived catalyst.

In order to further demonstrate the efficacy of the present invention and to provide a description of several other process steps which can make the IGCC process more flexible, the following examples were simulated. In these examples a base case without water addition has been run for each of the process configurations.

EXAMPLES

Example I

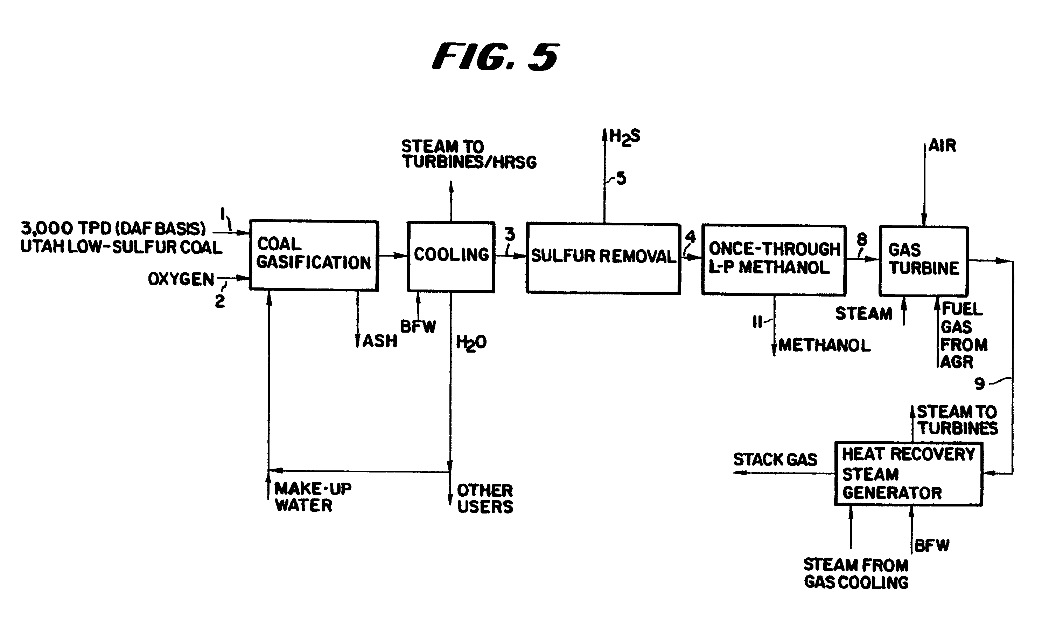

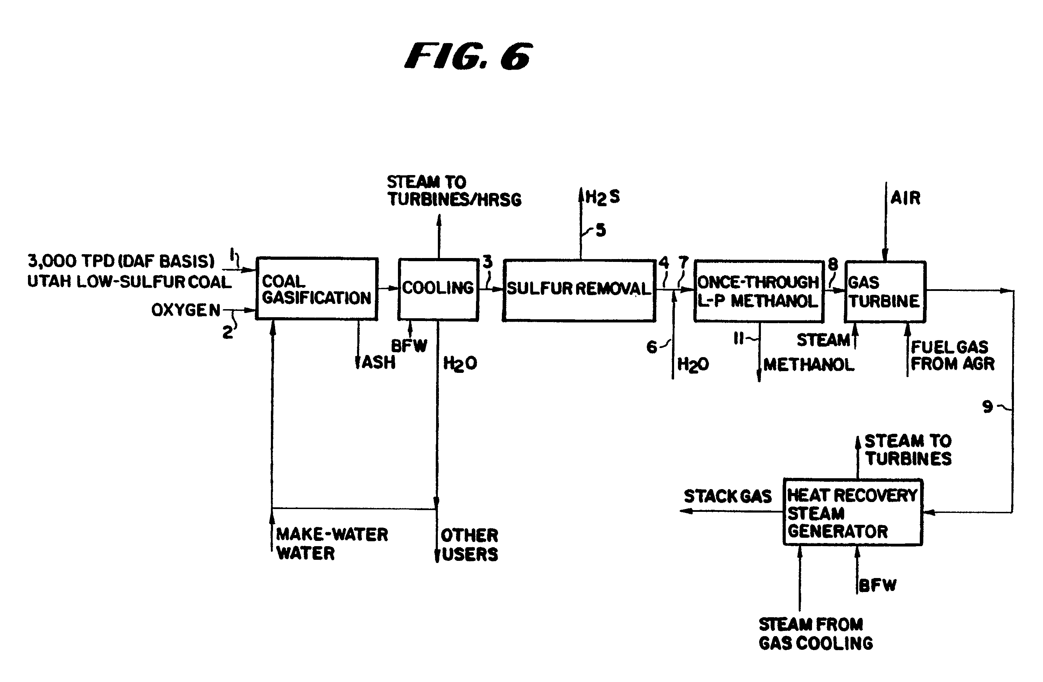

Figures 5 and 6 show block flow diagrams for a simple once-through liquid-phase methanol IGCC process. Figure 5 shows the process without water addition and Figure 6 with water addition. The corresponding material balances for 3,000 TPD of low sulfur coal for each figure are shown in Tables II and III, respectively.

TABLE II
IGCC LIQUID-PHASE METHANOL BASE CASE
FLOW RATES SHOWN ARE IN LBMOL/HR

| | STREAM NAME & NUMBER | | | | |
|---|---|---|---|---|---|
| COMPONENT | OXYGEN 2 | RAW GAS 3 | "CO-RICH" GAS 4 | ACID GAS 5 | FUEL GAS 8 |
| H2 | 0 | 8,648 | 8,645 | 3 | 4,638 |
| CO | 0 | 12,600 | 12,597 | 3 | 10,609 |
| CO2 | 0 | 4,482 | 3,211 | 1,271 | 3,108 |
| N2(CH4-Ar) | 173 | 409 | 247 | 162 | 247 |
| O2 | 8,459 | 0 | 0 | 0 | 0 |
| H2S | 0 | 287 | 0 | 287 | 0 |
| COS | 0 | 19 | 0 | 19 | 0 |
| H2O | 0 | 0 | 0 | 0 | 0 |
| CH3OH | 0 | 0 | 0 | 0 | 169 |
| TOTAL (#MPH) | 8,632 | 26,445 | 24,700 | 1,745 | 18,771 |
| TOTAL (LB/HR) | 275,878 | 590,472 | 518,700 | 71,772 | 455,906 |

| | STREAM NAME & NUMBER | |
|---|---|---|
| COMPONENT | TURBINE EXHAUST 9 | CRUDE METHANOL 11 |
| H2 | 0 | 0 |
| CO | 0 | 4 |
| CO2 | 14,023 | 89 |
| N2(CH4-AR) | 122,921 | 0 |
| O2 | 24,593 | 0 |
| H2S | 0 | 0 |
| COS | 0 | 0 |
| H2O | 12,952 | 14 |
| CH3OH | 0 | 1,828 |
| TOTAL (#MPH) | 174,489 | 1,935 |
| TOTAL (LB/HR) | 5,324,754 | 62,776 |

TABLE III
IGCC LIQUID-PHASE METHANOL WITH WATER ADDITION CASE
FLOW RATES SHOWN ARE IN LBMOL/HR

| | | STREAM NAME & NUMBER | | | |
| | OXYGEN | RAW GAS | "CO-RICH" GAS | ACID GAS | WATER |
| COMPONENT | 2 | 3 | 4 | 5 | 6 |
|---|---|---|---|---|---|
| H2 | 0 | 8,648 | 8,645 | 3 | 0 |
| CO | 0 | 12,600 | 12,597 | 3 | 0 |
| CO2 | 0 | 4,482 | 3,211 | 1,271 | 0 |
| N2(CH4-Ar) | 173 | 409 | 247 | 162 | 0 |
| O2 | 8,459 | 0 | 0 | 0 | 0 |
| H2S | 0 | 287 | 0 | 287 | 0 |
| COS | 0 | 19 | 0 | 19 | 0 |
| H2O | 0 | 0 | 0 | 0 | 2,139 |
| CH3OH | 0 | 0 | 0 | 0 | 0 |
| TOTAL (#MPH) | 8,632 | 26,445 | 24,700 | 1,745 | 2,139 |
| TOTAL (LB/HR) | 275,878 | 590,472 | 518,700 | 71,772 | 38,502 |

| | | STREAM NAME & NUMBER | | |
| | LPR INLET | FUEL GAS | TURBINE EXHAUST | CRUDE METHANOL |
| COMPONENT | 7 | 8 | 9 | 11 |
|---|---|---|---|---|
| H2 | 8,645 | 6,442 | 0 | 0 |
| CO | 12,597 | 8,349 | 0 | 3 |
| CO2 | 3,211 | 5,160 | 13,818 | 147 |
| N2(CH4-Ar) | 247 | 247 | 112,718 | 0 |
| O2 | 0 | 0 | 22,103 | 0 |
| H2S | 0 | 0 | 0 | 0 |
| COS | 0 | 0 | 0 | 0 |
| H2O | 2,139 | 1 | 14,659 | 42 |
| CH3OH | 0 | 177 | 0 | 1,972 |
| TOTAL (#MPH) | 26,839 | 20,376 | 163,298 | 2,164 |
| TOTAL (LB/HR) | 557,202 | 486,787 | 4,960,690 | 70,406 |

Example II

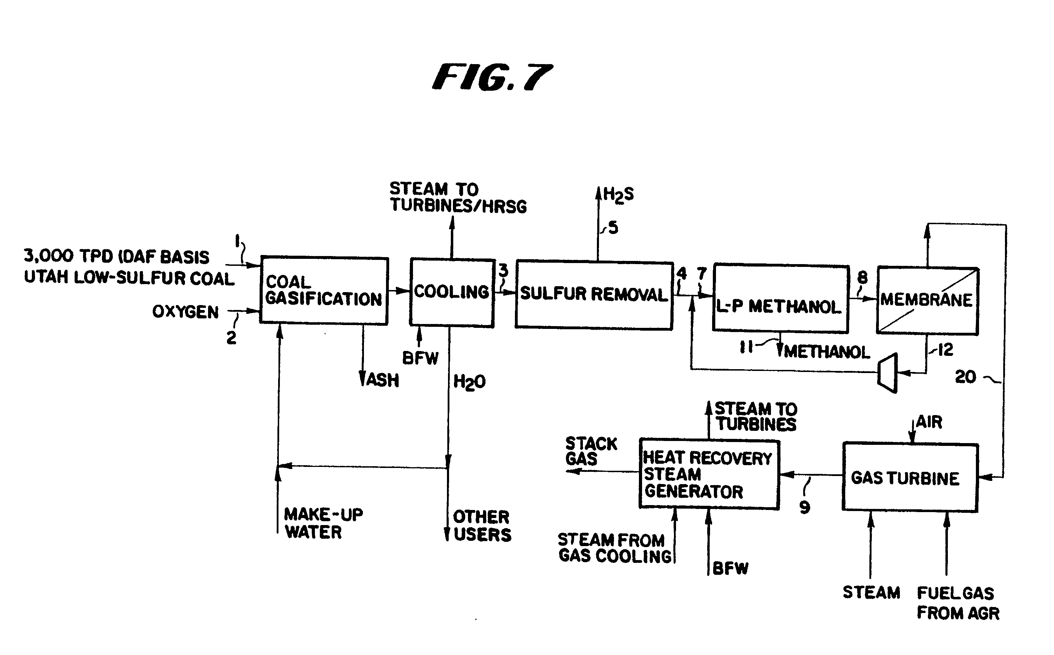

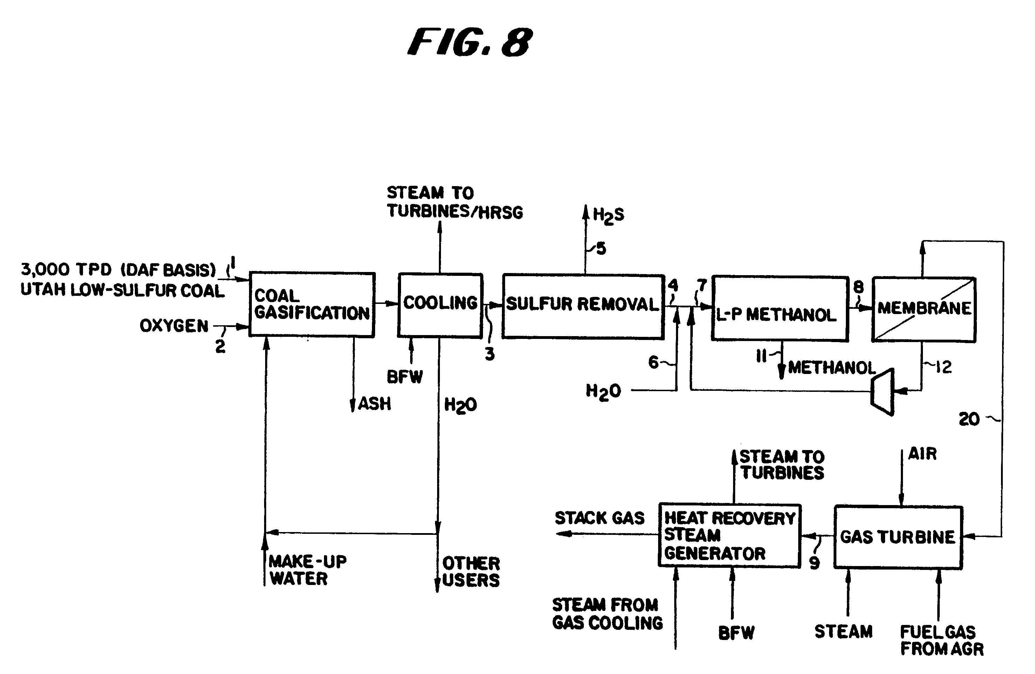

Figures 7 and 8 show block flow diagrams for a liquid-phase methanol IGCC process with a membrane recycle. Figure 7 shows the process without water addition and Figure 8 with water addition. The corresponding material balances for 3,000 TPD of low sulfur coal for each figure are shown in Tables IV and V, respectively.

It should be noted that the membrane material simulated in this example is a commercially available cellulose acetate. Other membranes with higher $H_2/CO_2$ selectivities will permit even greater increases in methanol production.

## TABLE IV
### IGCC LIQUID-PHASE METHANOL BASE CASE WITH MEMBRANE RECYCLE
### FLOW RATES SHOWN ARE IN LBMOL/HR

| | STREAM NAME & NUMBER | | | | |
|---|---|---|---|---|---|
| COMPONENT | OXYGEN 2 | RAW GAS 3 | "CO-RICH" GAS 4 | ACID GAS 5 | LPR INLET 7 |
| H2 | 0 | 8,648 | 8,645 | 3 | 12,858 |
| CO | 0 | 12,600 | 12,597 | 3 | 13,159 |
| CO2 | 0 | 4,482 | 3,211 | 1,271 | 5,267 |
| N2(CH4-Ar) | 173 | 409 | 247 | 162 | 256 |
| O2 | 8,459 | 0 | 0 | 0 | 0 |
| H2S | 0 | 287 | 0 | 287 | 0 |
| COS | 0 | 19 | 0 | 19 | 0 |
| H2O | 0 | 0 | 0 | 0 | 0 |
| CH3OH | 0 | 0 | 0 | 0 | 106 |
| TOTAL (#MPH) | 8,632 | 26,445 | 24,700 | 1,745 | 31,647 |
| TOTAL (LB/HR) | 275,878 | 590,472 | 518,700 | 71,772 | 637,029 |

| | STREAM NAME & NUMBER | | | | |
|---|---|---|---|---|---|
| COMPONENT | FUEL GAS 8 | MEMBRANE REJECT 20 | TURBINE EXHAUST 9 | MEMBRANE PERMEATE 12 | CRUDE METHANOL 11 |
| H2 | 7,021 | 2,809 | 0 | 4,213 | 0 |
| CO | 10,280 | 9,718 | 0 | 562 | 5 |
| CO2 | 5,023 | 2,963 | 12,864 | 2,056 | 183 |
| N2(CH4-Ar) | 256 | 247 | 97,782 | 9 | 0 |
| O2 | 0 | 0 | 19,504 | 0 | 0 |
| H2S | 0 | 0 | 0 | 0 | 0 |
| COS | 0 | 0 | 0 | 0 | 0 |
| H2O | 1 | 0 | 10,542 | 0 | 38 |
| CH3OH | 199 | 93 | 0 | 106 | 2,804 |
| TOTAL (#MPH) | 22,780 | 15,833 | 140,692 | 6,947 | 3,030 |
| TOTAL (LB/HR) | 536,960 | 418,631 | 4,313,373 | 118,329 | 98,598 |

### TABLE V
### IGCC LIQUID-PHASE METHANOL BASE CASE WITH MEMBRANE RECYCLE
### AND WATER ADDITION
### FLOW RATES SHOWN ARE IN LBMOL/HR

| | STREAM NAME & NUMBER | | | | |
|---|---|---|---|---|---|
| COMPONENT | OXYGEN 2 | RAW GAS 3 | "CO-RICH" GAS 4 | ACID GAS 5 | WATER 6 |
| H2 | 0 | 8,648 | 8,645 | 3 | 0 |
| CO | 0 | 12,600 | 12,597 | 3 | 0 |
| CO2 | 0 | 4,482 | 3,211 | 1,271 | 0 |
| N2(CH4-Ar) | 173 | 409 | 247 | 162 | 0 |
| O2 | 8,459 | 0 | 0 | 0 | 0 |
| H2S | 0 | 287 | 0 | 287 | 0 |
| COS | 0 | 19 | 0 | 19 | 0 |
| H2O | 0 | 0 | 0 | 0 | 2,139 |
| CH3OH | 0 | 0 | 0 | 0 | 0 |
| | | | | | |
| TOTAL (#MPH) | 8,632 | 26,445 | 24,700 | 1,745 | 2,139 |
| TOTAL (LB/HR) | 275,878 | 590,472 | 518,700 | 71,772 | 38,502 |

| | STREAM NAME & NUMBER | | | | | |
|---|---|---|---|---|---|---|
| COMPONENT | LPR INLET 7 | FUEL GAS 8 | MEMBRANE REJECT 20 | TURBINE EXHAUST 9 | MEMBRANE PERMEATE 12 | CRUDE METHANOL 11 |
| H2 | 15,175 | 10,882 | 4,353 | 0 | 6,530 | 0 |
| CO | 13,012 | 7,858 | 7,444 | 0 | 415 | 3 |
| CO2 | 6,536 | 8,220 | 4,893 | 12,533 | 3,325 | 268 |
| N2(CH4-Ar) | 256 | 256 | 247 | 85,659 | 9 | 0 |
| O2 | 0 | 0 | 0 | 16,656 | 0 | 0 |
| H2S | 0 | 0 | 0 | 0 | 0 | 0 |
| COS | 0 | 0 | 0 | 0 | 0 | 0 |
| H2O | 2,141 | 4 | 0 | 11,951 | 2 | 140 |
| CH3OH | 156 | 225 | 69 | 0 | 156 | 3,072 |
| | | | | | | |
| TOTAL (#MPH) | 37,275 | 27,445 | 17,009 | 126,799 | 10,436 | 3,483 |
| TOTAL (LB/HR) | 733,445 | 618,412 | 442,169 | 3,869,329 | 176,243 | 112,688 |

Example III

A series of laboratory experiments was carried out to compare the performance of the liquid phase methanol process using aromatic and paraffinic hydrocarbons respectively as the liquid medium. The experiments utilized two identical 50cc Microclave autoclave reactor systems in each of which a commercially-available methanol synthesis catalyst was slurried in powder form under agitation in the respective liquid medium. BASF S3-85 catalyst was selected for these experiments from among several commercially-available methanol synthesis catalysts, although any of these catalysts could have been used. One reactor was charged with the 100% paraffinic hydrocarbon oil Freezene 100 manufactured by the Witco Chemical Company, the properties of which are given in Table I. This is the same liquid used to obtain the data reported in Fig. 1 as determined in earlier studies. The second reactor was charged with the aromatic hydrocarbon oil Exxon Aromatic 150, manufactured by the Exxon Corporation; this oil consists primarily of $C_9$-$C_{11}$ aromatics, contains approximately 10 wt% naphthalene, and has a boiling range of 176-210° C. This is the same liquid used in the earlier-cited reference of Sherwin and Blum. In preparation for the experiments, the catalyst was reduced in situ using dilute synthesis gas. All experiments were carried out at the following conditions: pressure, 750 psig; temperature, 250°C; gas hourly space velocity (GHSV), 5000 std L/kg-hr; CO-rich feed gas composition of 35 mol% hydrogen, 51 mol% CO, 13 mol% $CO_2$, and 1 mol% $N_2$; and catalyst concentration in the hydrocarbon liquid, 20 wt%. Water was added as steam over the molar $H_2O/CO$ ratio range of 0 to 0.5. Methanol rate (productivity) was determined by material balance based on reactor effluent flow rates and gas compositions. The data points for the aromatic oil were obtained over a 10-hour period at increasing values of the $H_2O/CO$ ratio.

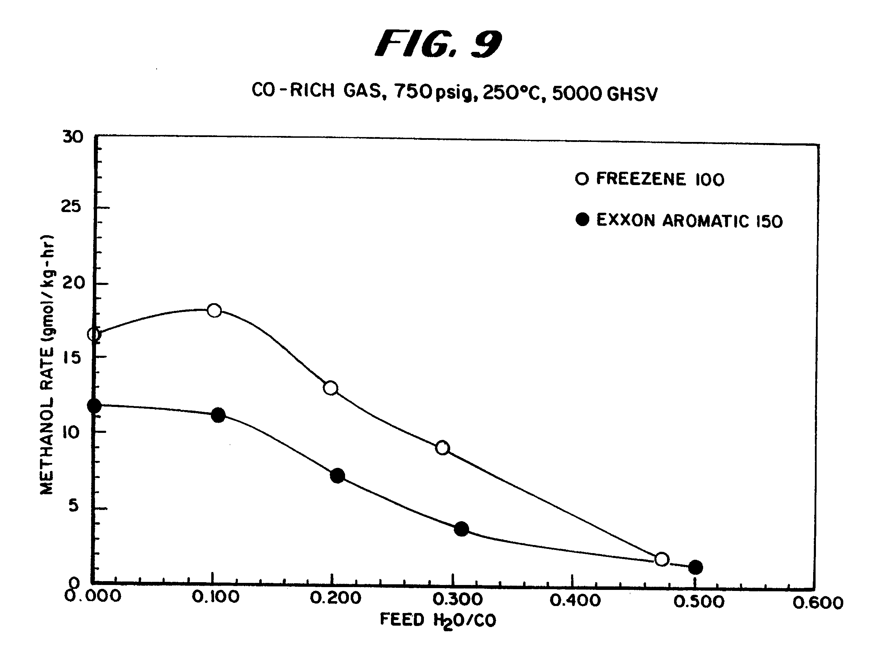

The results of these experiments are summarized in Fig. 9. The upper curve shows the effect of water addition on methanol rate (productivity) when the paraffinic Freezene 100 oil was used as the liquid medium. The data indicate that the methanol productivity is improved by the addition of water over a range of $H_2O/CO$ ratios, and is maximized at a ratio of about 0.1. These results are similar to those given in Fig. 1. The lower curve of Fig. 9 shows the effect of water addition when the aromatic oil Exxon Aromatic 150 was used as the liquid medium, and demonstrates that the methanol productivity decreases over the entire range of water addition tested, showing no improvement or maximum as seen in the upper curve for the paraffinic oil. These results as given by the lower curve are similar to the results of the earlier-cited Sherwin and Blum reference at p.5-6. The use of a paraffinic oil in the present invention thus is preferable over and distinctly different from the use of an aromatic oil as disclosed in this reference.

A separate set of experiments was carried out to investigate the reproducibility of the data determined for the use of the aromatic oil. A set of six runs was made using the same parameters and procedures described above except that the order and timing of the runs were varied. The results of the runs are given in Table VI.

### TABLE VI
### METHANOL RATE VS H2O/CO RATIO WITH WATER
### ADDITION FOR EXXON AROMATIC 150 LIQUID MEDIUM

| Run No. | H2/CO Molar Feed Ratio | Methanol Rate, gmoles/kg–hr |
|---------|------------------------|------------------------------|
| 1 | 0.0 | 12.75 |
| 2 | 0.098 | 11.94 |
| 3 | 0.0 | 8.27 |
| 4 | 0.098 | 6.2 |
| 5 | 0.29 | 0.97 |
| 6 | 0.47 | 0.46 |

Runs 1 and 2 were carried out over a period of about 2 hours and showed a decrease in methanol productivity similar to that of Fig. 9. The reactor was then idled for about three days by isolation under pressurized synthesis gas with agitation at about 240°C. Runs 3 and 4 were then carried out at the same conditions of Runs 1 and 2 over a period of about two hours, and points 5 and 6 were taken over an additional two hours. The results again indicate that methanol productivity decreases with increasing water addition, and also that a rapid catalyst deactivation occurs when this aromatic oil is used in this process. In numerous previous experiments using the paraffinic oil Freezene 100, no such catalyst deactivation was observed after similar reactor idling periods. While the mechanism of this catalyst deactivation in aromatic oil between runs with water addition is not understood, these experiments confirm that the use of paraffinic oils are preferred in the process of the present invention.

As can be seen from the Examples, the present invention includes several other process variations which add even more flexibility to the IGCC coproduction flowsheet. Figure 8 shows a proposed block flow diagram for a plant which incorporates a membrane loop into the effluent fuel gas stream to recover hydrogen for recycle to the liquid-phase reactor. The recycled hydrogen increases the feed $H_2/CO$ ratio to the reactor, which increases methanol production. The membrane can be used in conjunction with water addition to the liquid-phase methanol reactor, or without water addition. Mass and energy balances indicate that methanol production can be increased by 53% by using the membrane alone, and by an additional 15% by using both the membrane and water addition.

Table VII itemizes the relative methanol production which can be achieved in these various IGCC coproduct configurations. Clearly there is significant flexibility available through practicing this invention.

## TABLE VII
### RELATIVE METHANOL PRODUCTION FOR IGCC COPRODUCT
### PLANT VARIATIONS USING COMBINED SHIFT/SYNTHESIS

| Option | Methanol Production Compared to Option #1 |
|---|---|
| 1. Once Through Liquid-Phase Methanol | 100% |
| 2. With Water Addition | 108% |
| 3. With Membrane Recycle | 153% |
| 4. With Membrane Recycle and Water Addition | 168% |

The invention disclosed herein is an improved method for the production of methanol and fuel gas from CO-rich synthesis gas in a liquid phase reactor utilizing water addition to improve the reactor methanol productivity. The method can be combined with an Integrated Gasification Combined Cycle (IGCC) process for the efficient coproduction of methanol and electric power by combusting the fuel gas in a gas turbine which drives an electric generator. Methanol product can be used as supplemental fuel for the gas turbine during periods of peak power demand.

Important features of the invention include (1) the use of water addition to improve the methanol productivity of the liquid phase reactor, and (2) the use of the heat from the methanol synthesis reaction to improve the overall efficiency of power generation. This latter feature is accomplished by recovering heat from the liquid phase reactor effluent to raise steam, which is injected into the gas turbine to recover additional power. Heat can also be recovered directly from the reactor liquid medium by indirect heat exchange to generate additional steam for injection into the turbine.

The key advantage of the present invention is that methanol and electric power can be coproduced with a higher overall energy efficiency than that obtainable using separate conventional processes for the production of methanol and electric power.

The present invention has been described with reference to specific embodiments thereof. These embodiments should not be considered limitations on the scope of the present invention, the scope of which should be ascertained by the following claims.

### Claims

1. A method for producing methanol and fuel gas from synthesis gas rich in carbon monoxide comprising:
   (a) introducing said synthesis gas and water into a liquid phase reactor containing a solid catalyst and an inert paraffinic hydrocarbon liquid;
   (b) reacting said synthesis gas and water in the presence of said catalyst;
   (c) withdrawing from said reactor a reactor effluent stream comprising methanol, hydrogen, carbon monoxide, and carbon dioxide; and
   (d) cooling said reactor effluent stream and separating therefrom a methanol product and said fuel gas comprising hydrogen, carbon monoxide, and carbon dioxide.

2. The method of Claim 1 wherein the molar ratio of water to carbon monoxide introduced into said reactor is greater than zero and less than about 0.3, thereby improving methanol productivity over the productivity obtained when no water is introduced into said reactor.

3. The method of Claim 2 wherein said molar ratio of water to carbon monoxide is greater than zero and less than about 0.2.

4. The method of Claim 1 wherein the molar ratio R of said synthesis gas, where R is defined as $(H_2-CO_2)/(CO+CO_2)$, is less than about 2.0.

5. The method of Claim 1 wherein said water is introduced into said reactor as a liquid.

6. The method of Claim 1 wherein said methanol product and said fuel gas are separated from said reactor effluent stream by the steps of:
(a) cooling said reactor effluent stream and passing it into a first separator vessel, and withdrawing therefrom a first vapor stream and a first liquid stream;
(b) reducing the pressure of said first liquid stream and passing it into a second separator vessel, and withdrawing therefrom a second vapor stream and a second liquid stream; and
(c) heating and then expanding said first vapor stream to yield a third vapor stream;
wherein said fuel gas comprises said first and third vapor streams and said methanol product comprises said second liquid stream.

7. The method of Claim 1 wherein said solid catalyst comprises a methanol synthesis catalyst.

8. The method of Claim 7 wherein said solid catalyst further comprises a low temperature shift catalyst.

9. The method of Claim 1 wherein the concentration of said solid catalyst in said reactor is between about 5 and 50 weight percent.

10. The method of Claim 1 wherein a hydrogen-rich stream is separated from said fuel gas and recycled to said reactor.

11. The method of Claim 1 wherein said methanol product and electric power are coproduced in an integrated process comprising the additional steps of:
(a) generating a first stream of steam by indirect heat exchange between water and said reactor effluent stream, thereby cooling said reactor effluent stream;
(b) combusting said fuel gas with compressed air in a combustor of a gas turbine;
(c) injecting at least a portion of said first stream of steam into said combustor;
(d) withdrawing hot gas from said combustor and expanding the gas through an expander of said gas turbine; and
(e) utilizing said expander of said gas turbine to drive an electric generator thereby generating electric power.

12. The method of Claim 11 wherein heat is removed from said reactor by passing water through a cooling coil disposed within said reactor, thereby generating a second stream of steam.

13. The method of Claim 12 wherein at least a portion of said second stream of steam is injected into said combustor.

14. The method of Claim 11 wherein heat is removed from said reactor by withdrawing a portion of said paraffinic hydrocarbon liquid therefrom, cooling said portion by indirect heat exchange with water to generate steam, and returning said portion to said reactor.

15. The method of Claim 14 wherein at least a portion of said steam is injected into said combustor.

16. The method of Claim 11 wherein during periods of peak electric power demand at least a portion of said methanol product is combusted in said combustor with additional compressed air thereby increasing the amount of said electric power generated.

# FIG. 1

FIG.2

# FIG. 3

BALANCED GAS
750 psig, 250°C
5,000 GHSV

△ LIQUID PHASE; 300 C.C. AUTOCLAVE

MeOH PRODUCTIVITY g mole / kg·hr (y-axis)

TIME ON STREAM, HRS (x-axis)

EP 0 445 660 A2

## FIG. 4

MeOH PRODUCTIVITY
$\dfrac{\text{g mole}}{\text{Kg-hr}}$

CURVE # 1

CURVE # 2

WATER ON

OFF    ON    OFF    ON    OFF    ON    OFF

FOR WATER ON,
$H_2O/CO$
g moles/g moles, INLET = 0.108

CO - RICH GAS
900 psig 250 °C
5000 GHSV

HOURS ON SYNTHESIS GAS

20    18    16    14    12    10

0    20    40    60    80    100    120    140    160

# FIG. 5

STEAM TO TURBINES/HRSG

↑H₂S

AIR

3,000 TPD (DAF BASIS) UTAH LOW-SULFUR COAL — 1

OXYGEN — 2

COAL GASIFICATION → COOLING — 3 → SULFUR REMOVAL — 4 → ONCE-THROUGH L-P METHANOL — 8 → GAS TURBINE

↑ASH  BFW  H₂O

— 5

11 — METHANOL

STEAM ↑

FUEL GAS FROM AGR

↑MAKE-UP WATER

OTHER USERS

9

↑STEAM TO TURBINES

STACK GAS ← HEAT RECOVERY STEAM GENERATOR

STEAM FROM GAS COOLING   BFW

EP 0 445 660 A2

# FIG. 6

EP 0 445 660 A2

# FIG.7

EP 0 445 660 A2

# FIG. 8

STEAM TO
TURBINES/HRSG

H₂S

3,000 TPD (DAF BASIS)
UTAH LOW-SULFUR COAL

OXYGEN

COAL GASIFICATION

COOLING

SULFUR REMOVAL

L-P METHANOL

MEMBRANE

ASH

BFW

H₂O

H₂O

METHANOL

MAKE-UP WATER

OTHER USERS

STEAM TO TURBINES

AIR

STACK GAS

HEAT RECOVERY STEAM GENERATOR

GAS TURBINE

STEAM FROM GAS COOLING

BFW

STEAM

FUEL GAS FROM AGR

EP 0 445 660 A2

# FIG. 9

CO-RICH GAS, 750 psig, 250°C, 5000 GHSV

O FREEZENE 100

● EXXON AROMATIC 150

EP 0 445 660 A2